# EUROPEAN PATENT SPECIFICATION

(11) **EP 0 636 363 B1**
(45) Date of publication and mention of the grant of the patent: **23.05.2001**
(21) Application number: 94305499.9
(22) Date of filing: 26.07.1994
(51) Int. Cl.: A61K 9/127

(54) **Drug delivery system**
Verabreichungssystem für Arzneistoffe
Système pour la délivrance d'un médicament

(30) Priority: 27.07.1993 JP 18454893
(43) Date of publication of application: 01.02.1995
(73) Proprietor: TERUMO KABUSHIKI KAISHA, Tokyo 151 (JP)
(72) Inventor: Kawahara, Kazuo, c/o Terumo K. K., Ashigarakami-gun, Kanagawa 259-01 (JP); Uchiyama, Hideki, c/o Terumo K. K., Ashigarakami-gun, Kanagawa 259-01 (JP); Kimura, Junji, c/o Terumo K. K., Ashigarakami-gun, Kanagawa 259-01 (JP); Miyajima, Koichiro, Uji-shi, Kyoto (JP)
(74) Representative: Harrison, David Christopher

(56) References cited:
- EP-A- 0 028 917
- WO-A-88/07365
- GB-A- 2 218 096
- JOURNAL OF LIPOSOME RESEARCH, vol. 3, no. 2, July 1993, NEW YORK (US), pages 179-199, XP000322144 G.E. PLAUTZ ET AL.: "liposome mediated gene tranfer into vascular cells"
- JOURNAL OF LIPOSOME RESEARCH, vol. 3, no. 1, March 1993, NEW YORK (US), pages 51-70, XP002024504 L.S.S. GUO ET AL.: "cationic liposomes containing noncytotoxic phospholipid and cholesterol derivatives"

## Description

This invention relates to a drug carrier which is capable of being selectively accumulated on an injured portion of vascular endothelium, as well as capable of carrying therein a drug for diagnosing or treating that injured portion.

An injury to a blood vessel, in particular to endothelium of the aorta is generally known to give rise to an excessive proliferation of blood vessel smooth muscle cell, and to become one of the causes of hardening of the arteries (R. Ross et al, N. Engl. J. Med., 314,488 (1986)). This injury may be caused for example by the formation of a thrombus, or by a peroxidation reaction in the blood vessel. With the recent development of treatments for enlarging a constricted portion of coronary by methods such as physically pressing, cutting or burning the constricted portion, there has been noticed a new problem. This is the so-called restenosis, wherein the tissue injured by a physical operation as mentioned above thickenes, thereby constricting again a blood vessel which was enlarged by the physical operation.

This problem is very serious one because a method of enlarging a constricted coronary by percutaneously introducing a catheter into the blood vessel for physically pressing the constricted portion (PTCA) is now extensively utilized as a treatment for ischemic cardiac disease such as angina pectoris. Restenosis is now found in 30 to 50% of patients after this physical treatment, as prevention of restenosis has become very important to the overall success of the PTCA method.

Since this restenosis is found to be induced by an excessive proliferation of blood vessel smooth muscle cells an employment, a drug having the effect of inhibiting this proliferation has been sought. However, none of the drugs reported up to date to have exhibited satisfactory results in a clinical test, though a large number of drugs are reported to be effective in inhibiting the proliferation of smooth muscle cell which have been cultivated on a Petri dish (W. Cascells, Circulation, 86(3), 724 (1992)).

One of the reasons for this ineffectiveness of such drugs may be the fact that when these drugs are intravenously or orally administered to a living body generally, i.e. not locally, in an amount which would not badly affect the body as a whole, it is very difficult to cause such a sufficient density of these drugs to be accumulated on the injured region of endothelium of a blood vessel. For example, when heparin (which is effective in inhibiting the proliferation of smooth muscle cell) is to be administered to a living body generally, a sufficient dosage of heparin to bring about a satisfactory medical effect to the injured site would invite side reactions such as a tendency to bleeding. However, it has been confirmed by Okada et. al. that if heparin is administered concentratedly to the injured portion of a blood vessel, it is possible to inhibit the proliferation of smooth muscle cell without the side reaction (Okada et. al. (DDS), 7(1), 51, (1992)). Thus, Okada et. al. have confirmed that a sufficient increase in concentration of medicament at the injured portion of blood vessel and the resultant medical effect can be obtained by employing a method wherein a gel containing heparin is stuck on the outer surface of the blood vessel which has been injured, after performing thoracotomy.

However, this method of administrating heparin is too invasive, and it is considered inadmissible to employ such a method in combination with the PTCA medication method, since the practical value of the latter is that it is a low-invasive medication method.

There has been a consistent demand in the art to develop a drug carrier which is capable of selectively depositing a sufficient concentration of medicine on an injured portion of vascular endothelium, i.e. which is capable of being selectively accumulated there, as well as being capable of carrying therein a large amount of drug, even if the drug carrier is employed in a low invasive administration method, such as an oral or intravenous administration method, or a local administration method using a catheter at the utmost. However, there has been failure so far to find such a drug carrier.

There have been increased researches on the applicability of a closed vehicle such as a liposome, emulsion, lipid microsphere and nanoparticle as a drug carrier in a drug delivery system.

However, one of the most important problems in the employment of these closed vehicles as a drug carrier, there is the difficulty in controlling the closed vehicle in intracorporeal kinetics. In particular, the closed vehicle is easily caught by a reticuloendothelial system (RES) such as the liver or spleen. Therefore, it is very difficult to use the closed vehicle as a targeting medium for an organ other than RES. No attention has been paid to the use of a closed vehicle as a medium for targetting an injured portion of vascular endothelium.

When a closed vehicle is constructed of an ordinary membrane, it is very difficult to carry a drug in high concentration. As a result, the merits of the closed vehicle are vitiated or there is an increase in manufacturing cost. There is another problem that the closed vehicles tend to aggregate to each other through protein in the blood due to their poor stability in the blood, thereby making it difficult to actually utilize the closed vehicle as a drug carrier.

In order to overcome these problems, various proposals have been made; see: A method of improving the retentivity in blood of closed vehicle by using, as a membrane component, a charged material such as stearylamine (JP-A-63-77824), A method of modifying the surface of closed vehicle with a sugar such as sialic acid or glucuronic acid and their derivatives (Biochimica Biophysic Acta (BBA) 1126, 255 (1992), D.D.S, 7,345 (1992)), A method of using as a protein-adsorb inhibiting agent a hydrophilic polymer derivative such as polyethylene glycol (JP-A-2-149512 and JP-A-3-218309), and A method of using a glucosamine derivative (JP-A-4-159216).

However, none of these methods are successful in realizing a drug carrier which is capable of being selectively accumulated on an injured portion of vascular endothelium, as well as capable of carrying therein a large amount of drug.

The object of the present invention is to provide a drug carrier which is capable of carrying therein a drug at a high filling ratio, has good intracorporeal stability, and is capable of being selectively accumulated on an injured portion of vascular endothelium resulting for example from PTCA treatment or arterial sclerosis.

According to the present invention there is provided a drug carrier which is capable of being selectively accumulated on an injured portion of vascular endothelium, and comprises a compound having a region of positive charge at a physiological pH range which is exposed on the surface of the drug carrier, and a hydrophilic polymer derivative having a hydrophilic polymer region thereof exposed at least partially on the surface of the drug carrier.

The present inventors have found to their surprise in the course of studying the factors controlling intracorporeal kinetics of various drug carriers that if a drug carrier contains a compound having a positively charged portion in the physiological pH range, and at least a portion of the positively charged portion is exposed on the surface of the drug carrier, the drug carrier can be selectively accumulated on an injured portion of vascular endothelium.

They have also found to their surprise that if a hydrophilic polymer is present on the surface of the drug carrier in addition to the positively charged portion, it is possible, without harming the accumulation property of the drug carrier in relation to an injured portion of vascular endothelium, to prevent the drug carrier from being aggregated in blood, thereby making it possible to improve the stability in blood of the carrier, and also to prevent the drug carrier from being caught by the RES.

The carrier may be a fine particle such as a macromolecule, a microaggregate, a microglobule, a nanoglobule, a liposome and an emulsion.

The carrier may further comprise a phospholipid or derivatives thereof, and/or a lipid other than phospholipid and derivatives of the lipid, and/or a stabilizer, and/or anti-oxidant, and/or other surface-modifying agents.

The compound providing the positive charge may be a compound containing at least one kind of group selected from an aliphatic primary, secondary or tertiary amino group, an aliphatic quaternary ammonium group, an amidino group and an aromatic primary, secondary or tertiary amino group, an aromatic quaternary ammonium group, or from compounds comprising a residual group containing at least one hydroxyl group attached thereto in addition to an aliphatic primary, secondary or tertiary amino group, an aliphatic quaternary ammonium group, an amidino group and an aromatic primary, secondary or tertiary amino group, an aromatic quaternary ammonium group, or a basic amino acid derivative.

The compound may be an amino sugar, which may be a glucosamine, galactosamine, mannosamine, neuraminic acid, neuraminic acid ester, other kinds of monosaccharide, a free such monosaccharides, or a glycoside of such monosaccharide.

The drug for diagnosing or treating an injured portion of vascular endothelium may be a medicament which is an anti-inflammatory drug, an anti-cancer drug, an enzymatic drug, an antibiotic substance, an antioxidant, a lipid introjection drug, a hormone drug, an angiotensin transformation enzyme-inhibiting drug, a smooth muscle cell-proliferation/migration inhibitor, a platelet aggregation inhibitor, a release inhibitor for a chemical mediator, and a proliferation/migration inhibitor for vascular endothelium, preferably an X-ray contrast medium, a radioisotope marker labeled nuclear medical diagnostics or a nuclear magnetic resonance diagnostic drug.

The drug for diagnosing or treating an injured portion of vascular endothelium may be at least one kind of heparin, theophylline, a cyclic AMP(cAMP), captopril, papaverine and forskolin.

In the Drawings:
Fig. 1 depicts the retentivity in blood of liposome of this invention in comparison with a comparative liposome as they are tested in rats;
Fig. 2 depicts the accumulation ratio of liposomes of this invention at an injured portion of vascular endothelium in comparison with a comparative liposome as they are tested in rats;
Fig. 3 is a graph depicting changes with time of retentivity at an injured portion of vascular endothelium of liposome of this invention in comparison with a comparative liposome as they are tested in rats;
Fig. 4 is a graph depicting changes with time of retentivity at an injured portion of vascular endothelium of another liposome of this invention in comparison with a comparative liposome as they are tested in rats;
Fig. 5 depicts the accumulation ratio of liposomes of this invention to an injured portion of vascular endothelium in comparison with a comparative liposome as they are tested in hogs and rats;
Fig. 6 illustrates a relationship between a time interval beginning from the moment of injury to a blood vessel to the moment of administration and the accumulation ratio of liposomes of this invention to an injured portion of vascular endothelium;
Fig. 7 illustrates a method of calculating the thickening degree in the test 7;
Fig. 8 illustrates a test result of intracorporeal kinetics wherein the distribution of a drug carrier in a kinetics a graph illustrating an four-ply energy band element shown in Fig. 7; and
Fig. 9 illustrates a test result of intracorporeal kinetics wherein the accumulation ratio of the drug carrier of this invention at an injured portion of vascular endothelium is shown.

### Detailed Description of the Preferred Embodiment

This invention provides a drug carrier having, on its surface, a portion which is positively charged in the physiological pH range, and carrying a drug for diagnosing or treating an injured portion of vascular endothelium, the drug carrier being capable of being selectively accumulated on an injured portion of vascular endothelium. This invention further provides a drug carrier which contains on its surface a hydrophilic polymer in addition to the positively charged portion.

It is preferable that the drug carrier according to this invention has a particle diameter ranging from 0.02 to 250µm, more preferably from 0.05 to 0.4µm.

As to the structure of the drug carrier, various forms thereof are possible. However, it is preferable to select those which inherently have a capacity of carrying a high concentration of drug. Namely, a macromolecule, a microaggregate, a microglobule, a nanoglobule, a liposome, an emulsion, or a combination thereof.

The drug carrier of this invention is effective as long as it contains as an essential constituent a compound having a positively charged portion in a prescribed pH range, in particular in the physiological pH range and as long as it contains a hydrophilic polymer derivative having a hydrophilic polymer portion in addition to the compound having a positively charged portion. Other constituents may be selected from any other materials as far as the inclusion of the materials do not hinder the above basic structure. However, for safety and stability in a living body, it is preferable to employ as such a constituent a lipid or phospholipid or derivatives thereof, a stabilizer, anti-oxidant, or other surface-modifying agents.

Examples of the phospholipid include phosphatidyl choline (lecithin), phosphatidyl glycerol, phosphatidic acid, phosphatidylethanol amine, phosphatidyl inositol, sphingomyelin, cardiolipin, and other natural or synthetic phospholipid, or a hydrogenated product of any of these natural or synthetic phospholipids.

Examples of the stabilizer are sterol such as cholesterol - having an effect of lowering membrane fluidity - glycerol, and sugar such as sucrose.

Examples of the anti-oxidant are tocopherol and homologues, e.g. vitamin E.. Tocopherol includes four isomers, α β γ δ all of which are useful in this invention.

Examples of other surface modifying agents are the derivatives of water-soluble polysaccharides such as glucuronic acid, sialic acid and dextran.

As for the compound having a positively charged portion in the physiological pH range, any may be used as long as it does not hinder the structural stability of the drug carrier. However, it is preferable to employ compounds containing at least one aliphatic primary, secondary or tertiary amino group, aliphatic quaternary ammonium group, amidino group or aromatic primary, secondary or tertiary amino group, an aromatic quaternary ammonium group, or comprising a residual group containing at least one hydroxyl group attached thereto in addition to an aliphatic primary, secondary or tertiary amino group, an aliphatic quaternary ammonium group, an amidino group and an aromatic primary, secondary or tertiary amino group, an aromatic quaternary ammonium group.

Specifically, it is preferable to employ a derivative to be obtained from a reaction between the above-mentioned compounds and a hydrophobic compound such as a long chain aliphatic acid, e.g. palmitic acid or stearic acid, a long chain alcohol, a sterol, polyoxypropylene alkyl, or a glycerol aliphatic acid ester. These derivatives are effective in stably introducing a hydrophobic compound portion into the membrane of the drug carrier (for example liposome), and at the same time in arranging the portion positively charged in the physiological pH range on the surface of the drug carrier.

Specific examples of the compound having a positively charged portion at the physiological pH range are amino-sugar such as glucosamine, galactosamine, mannosamine, neuraminic acid, neuraminic acid ester, other kinds of monosaccharide, a free monosaccharides, and glycosides of these monosaccharides.

This amino-sugar is effective in stably introducing a hydrophobic compound portion into the membrane of the drug carrier (for example liposome), and at the same time in arranging the positively charged portion at the physiological pH range on the surface of the drug carrier.

In this invention, the term "physiological pH range" means the range of pH in a living body, in particular in blood, in cells, and in the interior of organella in the cellularity. Generally, the physiological pH range is in the range of pH 4 to pH 10, usually in the range of pH 6 to pH 8.

As for the hydrophilic polymer portion which is exposed on the surface of the drug carrier, any polymer may be used as long as it does not hinder the structural stability of the drug carrier. Examples of the hydrophilic polymer are polyethylene glycol, dextran, pullulan, ficoll, polyvinyl alcohol, styrene-maleic anhydride alternating copolymer, divinylether-maleic anhydride alternating copolymer, a synthetic polyamino acid, amylose, amylopectin, chitosan, mannan, cyclodextrin, pectin, and carrageenan. Among these compounds, polyethylene glycol is most preferable, since polyethylene glycol is very effective in improving retentivity in blood of the drug carrier.

When the hydrophilic polymer is employed in a form of a derivative incorporating therein a hydrophobic compound such as a long chain aliphatic acid, e.g. palmitic acid or stearic acid, a long chain alcohol, a sterol, polyoxypropylene alkyl, or a glycerol aliphatic acid ester, it is possible to stably introduce a hydrophobic compound portion into the membrane of the drug carrier (for example liposome), and therefore to dispose the hydrophilic polymer on the surface of the drug carrier.

Examples of the hydrophilic polymer derivative are polyethylene glycol phosphatidylethanol amine and polyethylene glycol cholesterol.

As a drug to be carried in the drug carrier, any kind of pharmaceutically allowable substances of pharmacologically active materials, physiologically active materials and diagnostic materials can be used depending on the purpose of diagnosing or treating an injured portion of vascular endothelium.

In view of the specific property of the drug carrier having a positive charge on the surface thereof, a neutral or anionic active substance may be more preferable in achieving a higher filling ratio of a drug, but subject to that drug can be employed as long as they are effective in controlling various reactions in the injured portion of vascular endothelium thereby to restore the injured portion to a normal blood vessel tissue, and they do not hinder the basic characteristics of the drug carrier. Examples of such drugs are anti-inflammatory, anti-cancer, enzymatic, antibiotic substance, antioxidant, lipid introjection, hormone, angiotensin transformation enzyme-inhibiting substances, smooth muscle cell-proliferation/migration inhibitor, a platelet aggregation inhibitor, a release inhibitor for a chemical mediator, and a proliferation/migration inhibitor for vascular endothelium.

In particular, a sulfated glycosaminoglycan such as heparin, an oligosaccharide and a polysaccharide or derivatives thereof are effective in inhibiting the proliferation/migration of a smooth muscle cell, which is known to be a cause of thickening of blood vessel at an injured portion of vascular endothelium. Other drugs useful in preventing the thickening of blood vessel are theophylline, a cyclic AMP(cAMP), captopril, papaverine and forskolin.

Since the drug carrier of this invention has the characteristic of being selectively accumulated on an injured portion of vascular endothelium, combinations of optimum drugs can be used depending on the features of the injured portion. It is known that, at the injured portion in particular, platelet activation is more likely to occur, and various kinds of leucocytes are accumulated thereon releasing various chemical mediators, thereby causing an inflammatory reaction. It is also known that the peroxidation and entrapment of lipid are likely to proceed in these tissues. It is considered that these reactions may proceed in a combined manner so as to cause an arteriosclerotic lesion. Since it is expected that the degree of influence of these phenomena on the lesion may differ depending on each case, any kinds of drugs useful for the treatment of these various symptoms can be employed in this invention.

It is important to identify an injured portion prior to the treatment with a drug. Therefore, any kinds of intracorporeal diagnostic drug such as an X-ray contrast medium, a radioisotope marker labeled nuclear medical diagnostics, or a nuclear magnetic resonance diagnostic drug can be conveniently carried in a drug carrier of this invention thereby making it possible to selectively identify an injured portion.

It is easily possible to obtain the drug carrier of this invention by means of conventional method. For example, a compound having a positively charged portion in a prescribed pH range, in particular in the physiological pH range, a phospholipid and other components for the drug carrier are mixed with an organic solvent such as chloroform in a flask, and then after removing through fractionation the organic solvent, the residue is dried in vacuum, thereby forming a thin film on the inner surface of the flask. Then, a drug is put into the flask, and the whole contents are violently agitated, thereby obtaining a liposome dispersion liquid. The resultant liposome dispersion liquid is then subjected to a centrifugal separation to form a supernatant liquid containing the portion of the drug which was left as being failed to be carried by the drug carrier. This supernatant liquid is then removed through decantation to obtain a dispersed liquid of the drug carrier. It is also possible to employ a high-pressure discharge emulsifier in which all of the components are mixed and the resultant mixture is discharged under a high pressure.

Although it is possible to administer the drug carrier of this invention through an intravenous injection and the like, it is preferable to utilize a catheter, i.e. the catheter is first introduced into a blood vessel so as to positioning the tip portion of the catheter near the injured portion of vascular endothelium, and then the drug carrier is delivered to the injured portion through the catheter.

The drug carrier of this invention is superior in retentivity in blood as well as on an injured portion of vascular endothelium, such that the drug carrier can be stably retained in blood for more than 6 hours, and on an injured portion of vascular endothelium for more than 3 days after the first administration.

It is desired in order to obtain a better effect that the drug carrier of this invention is administered to an injured portion of vascular endothelium within one hour after the moment of injury of blood vessel caused for example from the operation of PTCA. Because if the drug carrier is administered more than one hour later after the injury of blood vessel, the accumulation ratio of the drug carrier of this invention would be temporarily decreased due probably to the adhesion of platelet and others to the injured portion of vascular endothelium.

Accordingly, it is preferred to administer the drug carrier of this invention within one hour after the moment of injury of blood vessel.

However, when some period of time is lapsed after the injury, the accumulation ratio to an injured portion of vascular endothelium would be increased again. Therefore, the administration of the drug carrier more than one hour later after the injury of blood vessel is not ineffective, but is in fact effective.

This invention will be further explained with reference to the examples shown below.

### Example 1

### Synthesis of 6-o-palmitoyl-methyl-D-qalactosaminide

According to the conventional method, N-benzyloxycarbonyl-methyl-D-galactosaminide was obtained from D-galactosaminide. Then, 9.3g of N-benzyloxycarbonylmethyl-D-galactosaminide and 8ml of palmitoyl chloride were added to 50ml of pyridine, and the resultant mixture was then stirred under a nitrogen gas atmosphere for 24 hours. After the completion of reaction, the reaction mixture was poured into a 10% solution of ice-cooled hydrochloric acid. The reaction product was extracted with ethyl acetate, and resultant liquid extract was washed with a saturated sodium bicarbonate (NaHCO₃) and with a salt water respectively. After being dried with sodium sulfate anhydride (Na₂SO₄), the extract was subjected to a solvent-removing step to obtain a crude product, which was then recrystallized from an ethyl acetate solution to obtain 7.65g of N-benzyloxycarbonyl-6-o-palmitoyl-methyl-D-galactosaminide.

1.35g of this N-benzyloxycarbonyl-6-o-palmitoylmethyl-D-galactosaminide was dissolved in 50ml of methanol, and then a catalytic amount of 5% Pd-C was added to the solution to perform a catalytic reduction for 24 hours under normal temperature and pressure. Upon finishing the reaction, the reaction product was filtered to remove the solvent, and the resultant residue was refined with a silica gel column chromatography to obtain 874mg of the aimed compound of 6-o-palmitoyl-methyl-D-galactosaminide having a chemical structure (Formula I) shown below.

Data supporting this Formula 1 are shown below.
m. p. : 122-124°C

### Elemental analysis:

- Found value:: C=64.24%, H=10.84%, N=3.04%
- Calculated value:: C=64.00%, H=10.51%, N=3.25% (Calculated as C₂₃H₄₃O₆N)
- IR(KBr):: 3353cm⁻¹, 2917cm⁻¹, 2854cm⁻¹, 1728cm^{-1,} 1462cm⁻¹
- MS(FAB):: 435(M+1)⁺

¹H-NMR(DMSO-d₆) δ (ppm): 5.15 (n, 1H), 5.00 (n, 1H) 4.51 (d, J=3.4Hz, 1H), 4.30 (d, J=10.6Hz, 1H), 4.04 (dd, J=6.6Hz, J=6.8Hz, 1H), 3.53 (m, 1H), 3.26 (s, 3H), 3.10 (m, 2H), 2.40 (m, 1H), 2.29 (t, J=7.2Hz, 2H), 1.51 (m, 2H), 1.24 (b, 24H), 0.86 (t, J=6.0Hz, 3H).

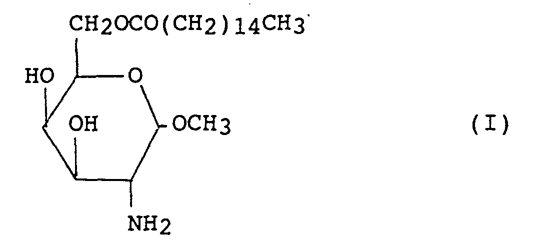

### Synthesis of liposome dispersion liquid

A liposome having, as a film-constituent component, 6-o-palmitoyl-methyl-D-galactosaminide polymer (PEG-PE) shown in Formula I was prepared as follows.

Three kinds of film-constituent components shown below, each being in the form of a chloroform solution, were charged into a 50ml bulb-shaped flask.
Phosphatidyl choline (Conc., 100mM): 840*µ*l
Cholesterol (Conc., 100mM): 240*µ*l
6-o-palmitoyl-methyl-D-galactosaminide (Conc., 10mM): 1200*µ*l

Thereafter, 10ml of chloroform was added thereto. After removing the chloroform through a fractionation, the mixture was dried overnight, thereby forming a lipid-like membrane on the inner wall of the flask. Then, 4ml of a 300mM sorbitol/10mM Tris-HCl buffer solution dissolving therein 100*µ*g of heparin was charged into the flask, and violently shaked and stirred thereby to obtain a liposome dispersion liquid (MLV).

The resultant dispersion liquid was then subjected to a centrifugal separation (12,000g, 70 minutes). The supernatant liquid containing heparin which had been failed to be carried in the carrier was then removed through decantation to obtain pellets of liposome. The pellets were then dispersed into a 300mM sorbitol/10mM Tris-HCl buffer solution to obtain an aimed liposome dispersion liquid containing therein heparin.

### Example 2

A liposome containing, as film-constituent components, 6-o-palmitoyl-methyl-D-galactosaminide and polyethylene glycol phosphatidylethanol amine shown in Formula II was prepared as follows.

Four kinds of film-constituent components shown below, each being in the form of a chloroform solution, were charged into a 50ml bulb-shaped flask.
Phosphatidyl choline (Conc., 100mM): 840*µ*l
Cholesterol (Conc., 100mM): 240*µ*l
6-o-palmitoyl-methyl-D-galactosaminide (Conc., 10mM): 1200*µ*l
PEG-PE (Conc., 0.4W/V%): 1000µl

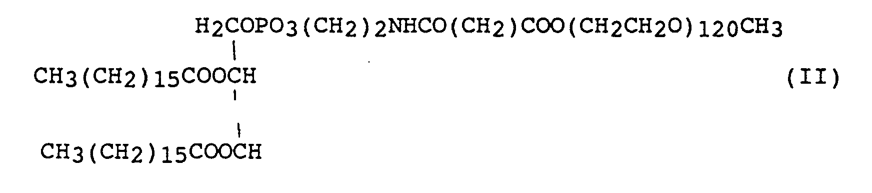

Thereafter, the same procedures were repeated as in Example 1, thereby obtaining an aimed liposome dispersion liquid having heparin carried therein.

### Example 3

### Synthesis of glucamine palmitate

### (1) N-Z modifying reaction of D-glucamine

3.62g (20.0mmol) of D-glucamine was dissolved into a mixed solvent consisting of 20ml of water and 20ml of tetrahydrofuran in a reaction vessel to obtain a mixed solution, to which 3.31g (24.0mmol) of potassium carbonate was dissolved. Then, the reaction vessel was maintained to a temperature of 0°C, and a solution of 2.86ml (20.0mmol) of benzyloxycarbonyl chloride (ZCl) in 10ml of tetrahydrofuran was dripped into the reaction vessel over 15 minutes. After stirring the reaction mixture for 3 hours, the precipitated portion was suction-filtered, washed twice with 3ml of tetrahydrofuran, and finally recrystallized using methanol chloroform as a recrystallization solvent thereby to obtain N-benzyloxycarbonyl glucamine.
- Yield:: 5.72g (about 90.8%)
- IR (νcm⁻¹) :: 3500-3200 (O-H), 3450 (N-H), 1650 (C=O)
- ¹H-NMR (δ ppm):: 3.3 (bs, N-CH₂- x2) 3.5-4.0 (m, HO-CH- x8) 5.1 (s, Ar-CH₂- x2) 7.9 (s, Ar-H- x5)

### (2) Primary selective esterification reaction using N-Z modified glucamine and palmitoyl chloride

3.15g (10.0mmol) of N-Z modified glucamine was dissolved into 50ml of pyridine to obtain a solution, to which 2.80g (10.2mmol) of palmitoyl chloride was slowly dripped over 10 minutes while agitating the solution under an ice-cooled condition. After 3 hours of reaction, the reaction mixture was diluted with 500ml of water, and neutralized with 4N hydrochloric acid. Then, the reaction product was extracted three times with 150ml of chloroform. Whole of the extracts were combined together, and washed with 100ml of a saturated salt water, and the resultant organic phase was dried over anhydrous sodium sulfate. After removing the solvent through fractionation, the residue was recrystallized using methanol chloroform as a recrystallization solvent thereby to obtain N-Z modified glucamine ester of palmitic acid. 1650 (C=O)
- Yield:: 4.63g (about 83.6%)
- IR (νcm⁻¹) :: 3500-3200 (O-H), 3450 (N-H), 1730 (C=O),
- ¹H-NMR (δ ppm):: 1.2 (s, -CH₂- x2) 3.3-4.2 (m, HO-CH- x8) 5.1 (s, Ar-CH₂- x2) 7.4 (s, Ar-H- x5)

### (3) Synthesis of glucamine palmitate

4.0g (7.23mmol) of N-Z modified glucamine ester of palmitic acid was dissolved into 100ml of a solvent (methanol:chloroform=10:1) to obtain a solution, to which 1g of palladium carbon (10%) (Nakaraitesk Co. Ltd.) was added to perform a hydrogenation overnight. Upon completion of the reaction, the reaction product was filtered and the solvent was removed through fractionation from the filtrate, thereby obtaining glucamine palmitate as shown in the following chemical formula III. 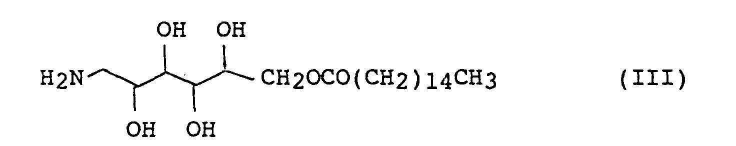
- Yield:: 4.63g (about 83.6%)
- IR (νcm⁻¹) :: 3500-3200 (O-H), 3380 (N-H), 3180 (N-H), 1730 (C=O)
- ¹H-NMR (δ ppm):: 1.2 (s, -CH₂- x2) 2.8 (bs, NH₂- x2)
3.3-4.2 (m, HO-CH- x8)

### Synthesis of liposome dispersion liquid containing glucamine palmitate as a membrane component

Three kinds of film-constituent components shown below, each being in the form of a chloroform solution, were charged into a 50ml bulb-shaped flask.
Phosphatidyl choline (Conc., 100mM): 840*µ*l
Cholesterol (Conc., 100mM): 240*µ*l
glucamine palmitate (Conc., 10mM): 1200*µ* l

Thereafter, 10ml of chloroform was added thereto. After removing the chloroform through a fractionation, the mixture was dried overnight, thereby forming a lipid-like membrane on the inner wall of the flask. Then, 4ml of a 300mM sorbitol/10mM Tris-HCl buffer solution dissolving therein 100*µ*g of heparin was charged into the flask, and violently shaked and stirred thereby to obtain a liposome dispersion liquid (MLV).

The resultant dispersion liquid was then subjected to a centrifugal separation (12,000g, 70 minutes). The supernatant liquid containing heparin which had been failed to be carried in the carrier was then removed through decantation to obtain pellets of liposome. The pellets were then dispersed into a 300mM sorbitol/10mM Tris-HCl buffer solution to obtain an aimed liposome dispersion liquid containing therein heparin.

### Example 4

A liposome having, as a film-constituent component, gulucaminepalmitoyl ester shown in Formula IV was prepared as follows.

Four kinds of film-constituent components shown below were charged, each being in the form of a chloroform solution, into a 50ml bulb-shaped flask.
Phosphatidyl choline (Conc., 100mM): 840*µ*l
Cholesterol (Conc., 100mM): 240*µ*l
Gulucaminepalmitoyl ester (Conc., 10mM): 1200*µ*l
PEG-PE (Conc., 0.4W/V%): 1000*µ*l

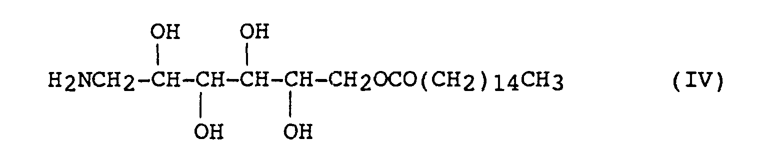

Thereafter, the same procedures were repeated as in Example 1, thereby obtaining an aimed liposome dispersion liquid having heparin carried therein.

### Example 5

A lysine derivative (L-lysylphosphatidylethanol amine) shown in Formula V was synthesized as explained below. A liposome containing, as a film-constituent component, the lysine derivative prepared above was manufactured as follows.

### Synthesis of a lysine derivative (L-lysylphosphatidylethanol amine)

L-lysylphosphatidylethanol amine to be used in this Example was prepared as follows. 1.05g of dipalmitoylphosphatidylethanol amine was dissolved into 50ml of methylene chloride to obtain a solution, to which 0.95g of N,N-dicarbobenzoxy-L-lysine available in the market and 1.46g of a water-soluble carbodi-imide were further added. The resultant mixture was then stirred for 24 hours at the room temperature. After the completion of reaction, the reaction mixture was poured into a 10% solution of ice-cooled hydrochloric acid to obtain a reaction product which was then extracted with ethyl acetate. Subsequently, the resultant liquid extract was washed with a saturated sodium bicarbonate (NaHCO₃) and with a salt water respectively. After being dried with sodium sulfate anhydride (Na₂SO₄), the extract was subjected to a solvent-removing step to obtain a crude product, which was then recrystallized from an ethyl acetate solution to obtain 1.51g of N,N-dicarbobenzoxy-L-lysyl-phosphatidylethanol amine.

1.45g of this N,N-dicarbobenzoxy-L-lysylphosphatidylethanol amine was dissolved in 50ml of methanol, and then a catalytic amount of 5% Pd-C was added to the solution to perform a catalytic reduction for 24 hours under normal temperature and pressure. Upon finishing the reaction, the reaction product was filtered to remove the solvent, and the resultant residue was refined with a silica gel column chromatography to obtain 865mg of the aimed compound of L-lysylphosphatidylethanol amine having a chemical structure (Formula V) shown below.

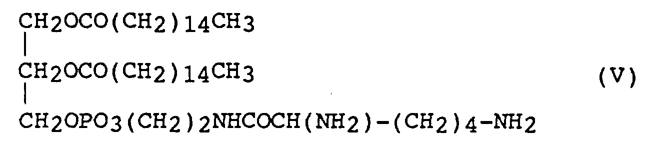

Four kinds of film-constituent components shown below were charged, each being in the form of a chloroform solution, into a 50ml bulb-shaped flask.
Phosphatidyl choline (Conc., 100mM): 840*µ*l
Cholesterol (Conc., 100mM): 240*µ*l
A lysine derivative (L-lysilphosphatidylethanol amine) (Conc., 10mM): 1200*µ*l
PEG-PE (Conc., 0.4W/V%): 1000*µ*l

Thereafter, the same procedures were repeated as in Example 1, thereby obtaining an aimed liposome dispersion liquid having heparin carried therein.

### Example 6

An arginine derivative (L-arginylphosphatidylethanol amine) shown in Formula VI was synthesized as explained below. A liposome containing, as a film-constituent component, the arginine derivative prepared above was manufactured as follows.

### Synthesis of an arginine derivative (L-arginylphosphatidylethanol amine)

L-arginyl-phosphatidylethanol amine to be used in this Example was prepared as follows. 1.03g of dipalmitoylphosphatidylethanol amine was dissolved into 50ml of methylene chloride to obtain a solution, to which 0.64g of N-carbobenzoxy-N-nitro-L-arginine available in the market and 2.6ml of iso-butylchloroformate were further added. The resultant mixture was then stirred for 5 hours at -15°C. After the completion of reaction, the reaction mixture was poured into a 10% solution of ice-cooled hydrochloric acid to obtain a reaction product which was then extracted with ethyl acetate. Subsequently, the resultant liquid extract was washed with a saturated sodium bicarbonate (NaHCO₃) and with a salt water respectively. After being dried with sodium sulfate anhydride (Na₂SO₄), the extract was subjected to a solvent-removing step to obtain a crude product, which was then recrystallized from an ethyl acetate solution to obtain 1.34g of N-carbobenzoxy-N-nitro-L-arginyl-phosphatidylethanol amine.

1.26g of this N-carbobenzoxy-N-nitro-L-arginylphosphatidylethanol amine was dissolved in 50ml of methanol, and then a catalytic amount of 5% Pd-C was added to the solution to perform a catalytic reduction for 24 hours under normal temperature and pressure. Upon finishing the reaction, the reaction product was filtered to remove the solvent, and the resultant residue was refined with a silica gel column chromatography to obtain 847mg of the aimed compound of L-arginyl-phosphatidylethanol amine having a chemical structure (Formula VI) shown below.

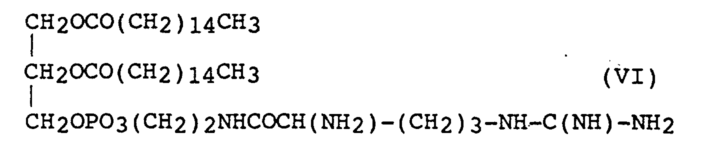

Four kinds of film-constituent components shown below, each being in the form of a chloroform solution, were charged into a 50ml bulb-shaped flask.
Phosphatidyl choline (Conc., 100mM): 840*µ*l
Cholesterol (Conc., 100mM): 240*µ*l
An arginine derivative (arginylphosphatidylethanol amine) (Conc., lOmM): 1200*µ*l
PEG-PE (Conc., 0.4W/V%): 1000*µ*l

Thereafter, the same procedures were repeated as in Example 1, thereby obtaining an aimed liposome dispersion liquid having heparin carried therein.

### Example 7

### Synthesis of N-palmitoyl-3-[(3-aminopropoxy)-poly(oxyethylene)] propylamine (molecular weight: 4000)

4.0g (about 1.0mmol) of Sanbright VFM-5000 (average molecular weight: about 4000, a product of Nihon Yushi Co. Ltd.) was dissolved into 150ml of chloroform, and the resultant solution was charged into a flask. Then, 0.14ml (1.0mmol) of triethylamine was further charged into the flask, and further 0.30g (1.0mmol) of a solution of palmitoyl chloride in 30ml of chloroform was dripped over 5 minutes by using a dropping funnel while stirring the whole mixture under an ice-cooled condition.

Thereafter, the whole mixture was heated up to 80°C, and the reaction was continued for 16 hours. Upon completing the reaction, 300ml of ice water was added to the reaction mixture to dilute the reaction mixture, which was then extracted three times with 100ml of methylene dichloride. Whole of the extracts were then combined together, and washed with 100ml of a saturated salt water, and the resultant organic phase was dried over anhydrous sodium sulfate. After the filtration thereof, the solvent was removed through a fractionation under a reduced pressure, the oily residue was refined by means of gel-filtration thereby to obtain N-palmitoyl-3-[(3-aminopropoxy)-poly(oxyethylene)] propylamine (molecular weight: 4000) having a formula VII shown below.
- Yield:: 1.84g (about 43.4%)
- IR (νcm⁻¹) :: 3500 (N-H), 2860 (C-H), 1670 (C=O), 1100 (C-O),
- ¹H-NMR (δ ppm):: 1.26 (S, -CH₂-)
3.63 (S, O-CH2-)

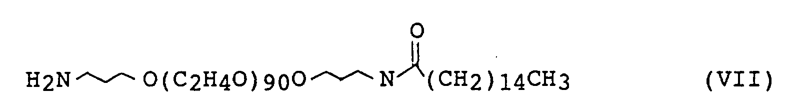

### Synthesis of liposome dispersion liquid containing N-palmitoyl-3-[(3-aminopropoxy)-poly(oxyethylene)] propylamine (molecular weight: 4000) as a membrane component

Three kinds of film-constituent components shown below were charged, each being in the form of a chloroform solution, into a 50ml bulb-shaped flask.
Phosphatidyl choline (Conc., 100mM): 840µl
Cholesterol (Conc., 100mM): 240µl
N-palmitoyl-3-[(3-aminopropoxy)-poly(oxyethylene)] propylamine (molecular weight: 4000) (Conc., 10mM): 1200µl

Thereafter, 10ml of chloroform was added thereto. After removing the chloroform through a fractionation, the mixture was dried overnight:, thereby forming a lipid-like membrane on the inner wall of the flask. Then, 4ml of a 300mM sorbitol/10mM Tris-HCl buffer solution dissolving therein 100*µ*g of heparin was charged into the flask, and violently shaked and stirred thereby to obtain a liposome dispersion liquid (MLV).

The resultant dispersion liquid was then subjected to a centrifugal separation (120,000g, 70 minutes). The supernatant liquid containing heparin which had been failed to be carried in the carrier was then removed through decantation to obtain pellets of liposome. The pellets were then dispersed into a 300mM sorbitol/10mM Tris-HCl buffer solution to obtain an aimed liposome dispersion liquid containing heparin therein.

### Example 8

### Synthesis of N-palmitoyl-3-[(3-aminopropoxy)-poly(oxyethylene)] propylamine (molecular weight: 2000)

2.0g (about 1.0mmol) of Sanbright VFM-5001-20 (average molecular weight: about 2000, a product of Nihon Yushi Co. Ltd.) was dissolved into 150ml of chloroform, and the resultant solution was charged into a flask. Then, 0.14ml (1.0mmol) of triethylamine was further charged into the flask, and further 0.30g (1.0mmol) of a solution of palmitoyl chloride in 30ml of chloroform was dripped over 5 minutes by using a dropping funnel while stirring the whole mixture under an ice-cooled condition.

Thereafter, the whole mixture was heated up to 80°C, and the reaction was continued for 16 hours. Upon completing the reaction, 300ml of ice water was added to the reaction mixture to dilute the reaction mixture, which was then extracted three times with 100ml of methylene dichloride. Whole of the extracts were then combined together, and washed with 100ml of a saturated salt water, and the resultant organic phase was dried over anhydrous sodium sulfate. After the filtration thereof, the solvent was removed through a fractionation under a reduced pressure, the oily residue was refined by means of gel-filtration thereby to obtain N-palmitoyl-3-[(3-aminopropoxy)-poly(oxyethylene)] propylamine (molecular weight: 2000) having a formula VIII shown below.
- Yield:: 0.92g (about 41.0%)
- IR (νcm⁻¹) :: 3500 (N-H), 2860 (C-H), 1670 (C=O), 1100 (C-O),
- ¹H-NMR (δ ppm):: 1.25 (S, -CH2-) 3.62 (S, O-CH2-)

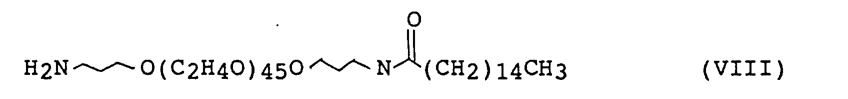

### Synthesis of liposome dispersion liquid containing N-palmitoyl-3-[(3-aminopropoxy)-poly(oxyethylene)] propylamine (molecular weight: 2000) as a membrane component

Three kinds of film-constituent components shown below were charged, each being in the form of a chloroform solution, into a 50ml bulb-shaped flask.
Phosphatidyl choline (Conc., 100mM): 840*µ* l
Cholesterol (Conc., 100mM): 240*µ*l
N-palmitoyl-3-[(3-aminopropoxy)-poly(oxyethylene)] propylamine (molecular weight: 2000) (Conc., 10mM): 1200*µ*l

Thereafter, the same procedures are repeated as in Example 7, thereby to obtain a liposome dispersion liquid (MLV).

### (Test 1: Measurements of filling ratio of heparin)

The filling ratio of heparin in a liposome in each of the Examples 1 to 8 was calculated as follows.

First the amount of heparin in the supernatant liquid obtained as a result of centrifugal separation was determined thereby determining the amount of heparin that had been failed to be filled into the liposome. This amount of heparin was then compared with the total amount of the heparin employed in the beginning for preparing the liposome, thus calculating the filling ratio of heparin.

For the purpose of comparison, a neutral liposome, as well as a liposome dispersion solution which does not contain either a compound having a positively charged portion in the physiological pH, or a hydrophilic polymer derivative were prepared in the same manner as in the above-mentioned Examples in addition to the liposome dispersion solutions which contain either a compound having a positively charged portion in the physiological pH, or both of a compound having a positively charged portion in the physiological pH and a hydrophilic polymer derivative. The filling ratio of this neutral liposome dispersion liquid was also measured. The results of the measurements are shown in Table 1 shown below.

**Table 1**

| Liposome dispersion liquid | Filling ratio (%) |
|---|---|
| Ex. 1 (Galactosamine) | 46.2 |
| Ex. 2 (Galactosamine+PEG-PE) | 44.9 |
| Ex. 4 (Glucamine+PEG-PE) | 43.2 |
| Ex. 5 (lysine+PEG-PE) | 45.3 |
| Ex. 6 (Arginine+PEG-PE) | 47.2 |
| Ex. 3 (Glucamine) | 48.3 |
| Ex. 7 (PEG+NH₂ (4000)) | 45.2 |
| Ex. 8 (PEG+NH₂ (2000)) | 46.2 |
| Comp. Ex. (Neutral liposome) | 17.7 |

As apparent from the above results, liposomes which contain either a compound having a positively charged portion in the physiological pH, or both of the compound having a positively charged portion in the physiological pH and a hydrophilic polymer derivative indicated a higher filling ratio as compared with the neutral liposome.

Although not shown in the Table 1, the liposomes containing a compound having a positively charged portion in the physiological pH other than above-mentioned compound also indicated almost the same excellent filling ratio as those of the Examples.

### (Test 2: Intracorporeal kinetics)

500µl of the liposome dispersion liquid containing 100mM of carboxyfluorescein prepared in accordance with Examples 1 and 2 was intravenously administered through a femoral vein to an SD male rat anesthetized with urethane. Blood samples were periodically taken out of the rat, and kept in an Eppendorf tube. The sampled blood was then measured for a fluorescence intensity thereby investigating the intracorporeal kinetics.

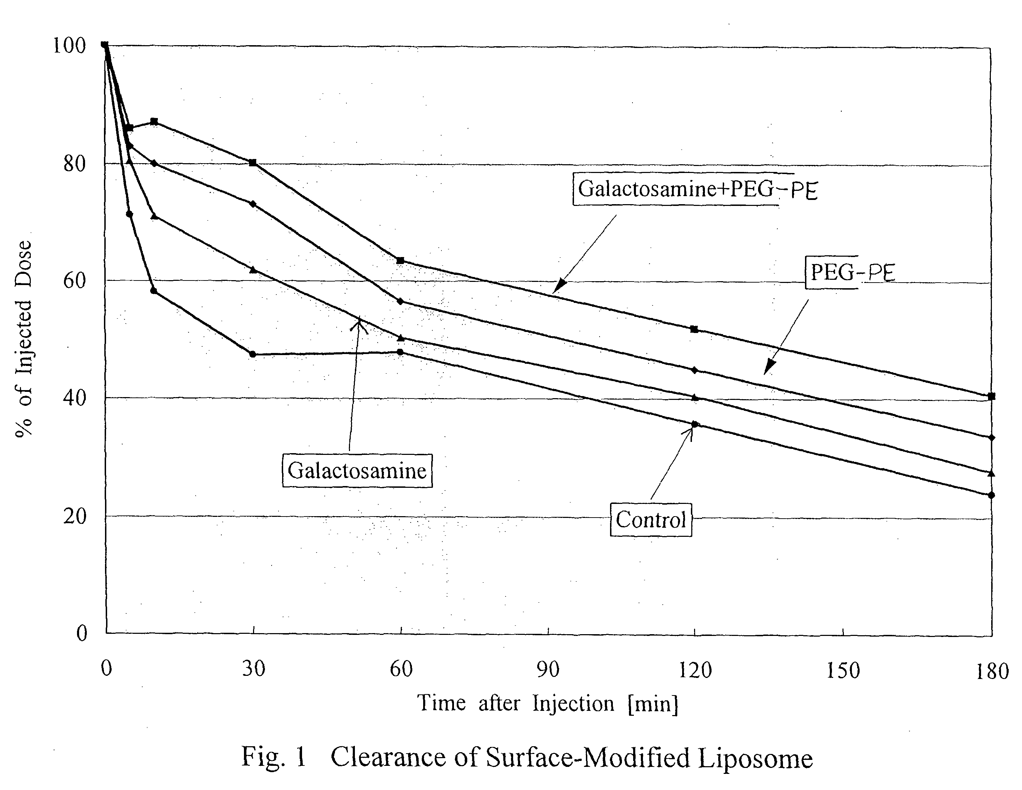

For the purpose of comparison, the same measurements were also conducted on the liposome dispersion liquid which was prepared in accordance with the above Examples without employing 6-o-palmitoyl-methyl-D-galactosaminide and PEG-PE, as well as on the liposome dispersion liquid which was surface-modified only with polyethylene glycol phosphatidyl ethanolamine (PEG-PE). Results are shown in Fig 1.

As apparent from Fig. 1, the liposomes of this invention which contained as a membrane component a compound having a positively charged portion at the physiological pH were recognized as being higher in retentivity in blood as compared with the liposome which does not contain such a compound. Further, the liposomes which were surface-modified with a hydrophilic polymer (PEG-PE) were recognized as being still higher in retentivity in blood as compared with the liposome which contained only a compound having a positively charged portion at the physiological pH. Further, the liposomes which were surface-modified with a hydrophilic polymer (PEG-PE) indicated a higher retentivity in blood as compared with the liposome which was surface-modified with only PEG.

Although not shown in the Fig 1, the liposomes containing a compound having a positively charged portion in the physiological pH other than above-mentioned compound also indicated almost the same excellent filling ratio as those of the Examples.

### (Test 3: Distribution in organs 1

A balloon catheter of 2-French size was introduced through a femoral vein into an SD male rat anesthetized with urethane. Then, the carotid artery of the rat was denuded 5 times thereby forming a model of injured vascular endothelium.

Immediately after this denudation, 500*µ*l of the liposome dispersion liquid containing 100mM of carboxyfluorescein prepared respectively in accordance with Examples 1 to 8 was intravenously administered through a femoral vein to the SD male rat. The samples of the carotid artery were taken out on the third hour, 16th hour, 24th hour and third day. The carboxyfluorescein was extracted from the carotid artery thus taken out, and the fluorescence intensity thereof was measured thereby estimating the accumulation volume of the liposome on the carotid artery.

Likewise, 500*µ*l of the liposome dispersion liquid containing 100mM of carboxyfluorescein prepared respectively in accordance with each of Examples 1 to 8 was intravenously administered to the SD male rat through a femoral vein which was not subjected to denudation. Thereafter, the distribution of the liposome to the carotid artery was measured in the same manner as explained above.

For the purpose of comparison with the liposomes of Examples 1 to 8, a liposome dispersion solution (Control) which does not contain either a compound having a positively charged portion in the physiological pH, or a hydrophilic polymer derivative was also prepared in the same manner as in the above-mentioned Examples. The amount of the accumulation of liposome of this control liposome dispersion liquid to the carotid artery representing a model of injured vascular endothelium was also measured.

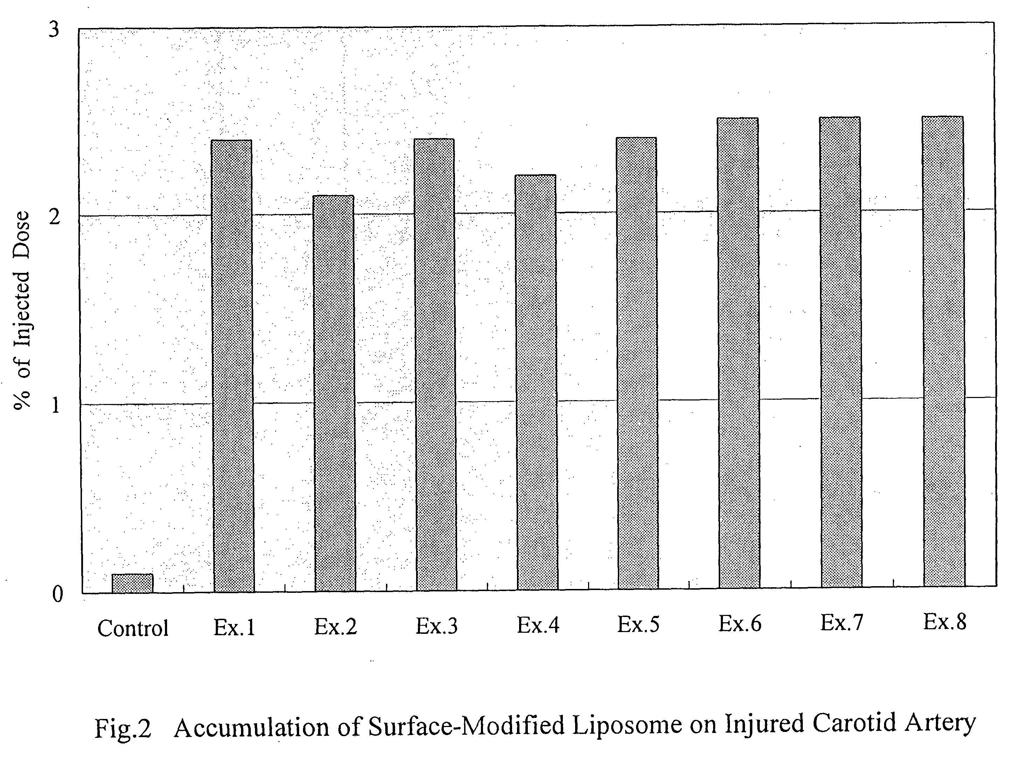

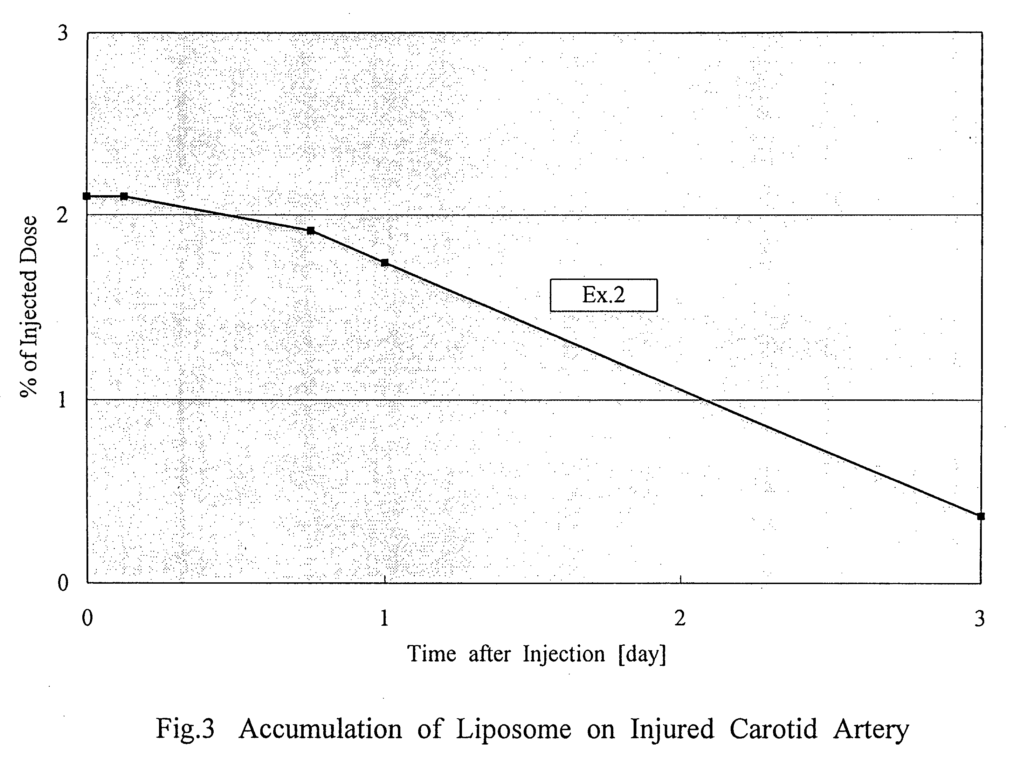

The results of the measurements which were measured on the third hour about the carotid artery treated with the liposome dispersion liquid of each of the Examples 1 to 8 and the Control are shown in Fig. 2, and the results of the measurements which were measured on the third day are shown in Fig. 3. Although not shown in these Figs., the accumulation volume of liposome of each of Examples 1 to 8 on a vascular endothelium which was not injured was almost the same as that of the Control liposome dispersion liquid.

As apparent from these results, the liposomes of this invention which contained as a membrane component a compound having a positively charged portion at the physiological pH, or which contained a hydrophobic residue and a hydrophilic polymer derivative in addition to the compound having a positively charged portion were recognized as being higher in accumulation ratio to an injured portion of blood vessel as compared with the liposome which does not contain any of such compounds. Further, since the accumulation ratio of the liposomes of this invention was higher at the injured portion of blood vessel as compared with that at the normal portion of blood vessel which was not injured, the liposomes of this invention was proved to be excellent in targeting property.

Further, it was confirmed that the liposomes of this invention could be retained on the injured portion of the blood vessel for 3 days if the liposome was administered immediately after the denudation.

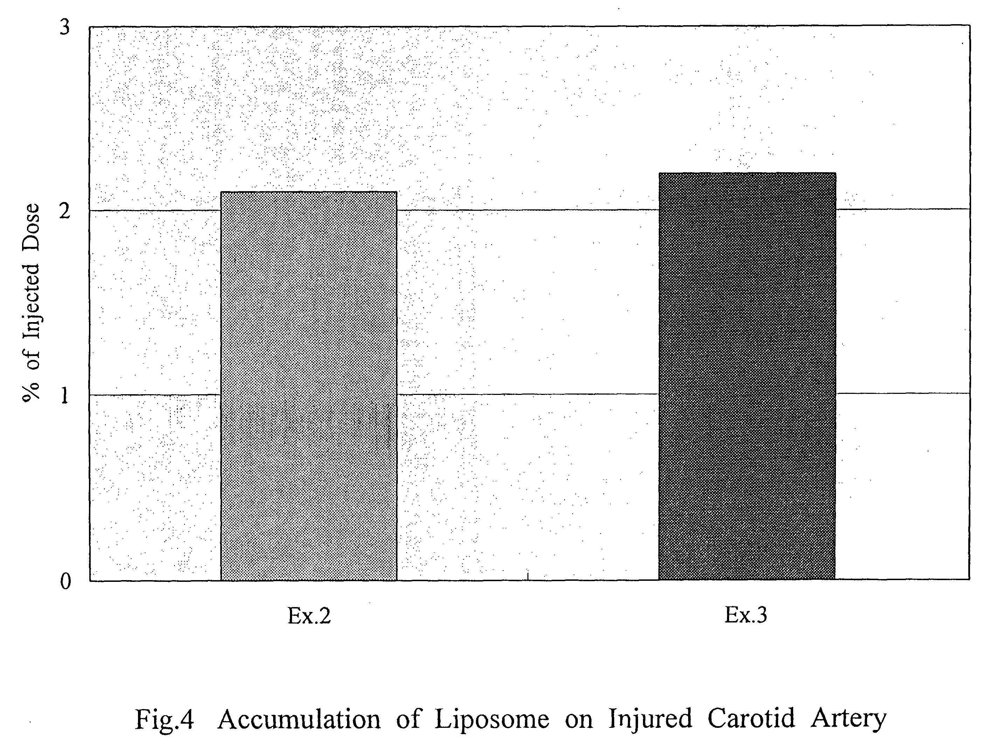

When the same test using a rat was conducted on the liposomes which were prepared in accordance with Examples of 2 and 4, excellent results as those obtained in other examples were obtained as shown in Fig. 4.

Although not shown in these Figs., the liposomes containing a compound having a positively charged portion in the physiological pH other than above-mentioned compounds also indicated almost the same excellent results as those of the Examples 2 and 4.

### (Test 4: Intracorporeal kinetics 2)

The same experiment as in the Test 2 was repeated except that a hog (30kg) was employed in place of a rat. Namely, 30ml of the liposome dispersion liquid containing 100mM of carboxyfluorescein prepared in accordance with Example 2 was intravenously administered through a femoral vein to the hog. The samples of blood were periodically taken out. The plasma was extracted from the blood thus taken out, and the fluorescence intensity thereof was measured thereby estimating the intracorporeal kinetics.

For the purpose of comparison, the same measurements were also conducted on the liposome dispersion liquid which was prepared in accordance with the above Example 2 without employing 6-o-palmitoyl-methyl-D-galactosaminide and PEG-PE. As a result, the liposome dispersion liquid of this invention showed an excellent retentivity in blood as in the case of Test 2, thus demonstrating the effectiveness even to a hog. Further, the Control was also indicated the same tendency as in the case of Test 2.

### (Test 5: Distribution in organs 2

A balloon catheter of 7.5-French size was introduced through a femoral artery into a hog (30kg) anesthetized with urethane. Then, the carotid artery of the hog was denuded 5 times thereby forming a model of injured vascular endothelium.

After this denudation, 30ml of the liposome dispersion liquid containing 100mM of carboxyfluorescein prepared respectively in accordance with Example 2 was intravenously administered through a femoral vein to the hog. The samples of the carotid artery were taken out on the third hour. The carboxyfluorescein was extracted from the carotid artery thus taken out, and the fluorescence intensity thereof was measured thereby estimating the distribution of the liposome on the carotid artery.

Likewise, 500*µ*l of the liposome dispersion liquid containing 100mM of carboxyfluorescein prepared respectively in accordance with Examples 1 and 2 was intravenously administered to the hog through a femoral vein which was not subjected to denudation. Thereafter, the distribution of the liposome to the carotid artery was measured in the same manner as explained above.

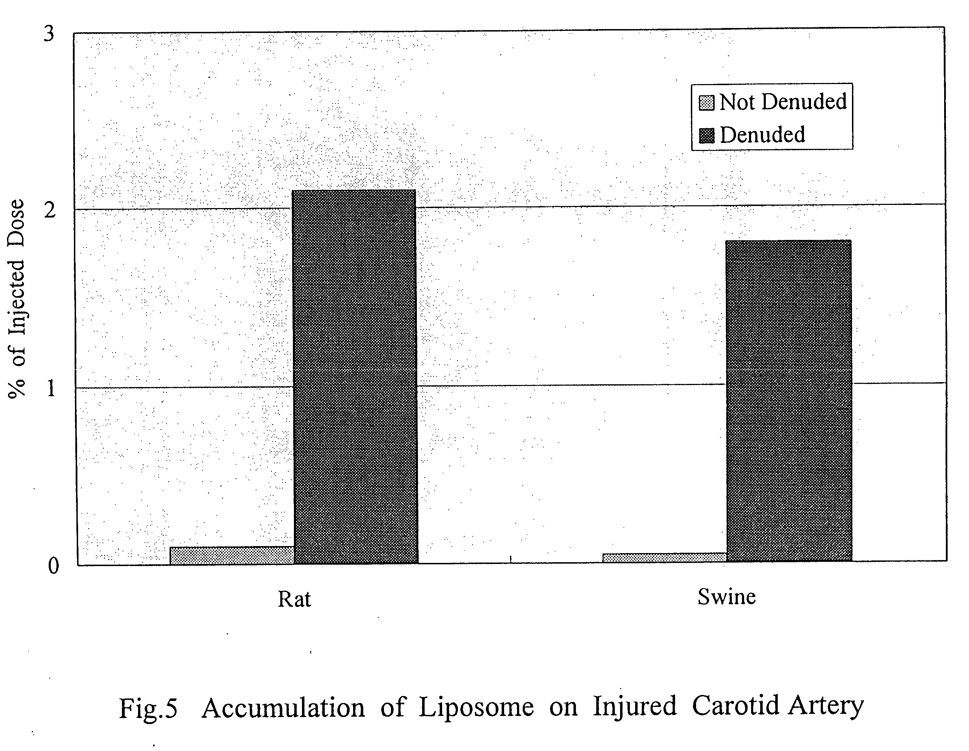

For the purpose of comparison, the same measurements were also conducted on the liposome dispersion liquid which was prepared in accordance with the above Examples but without employing 6-o-palmitoyl-methyl-D-galactosaminide and PEG-PE. The results are shown in Fig. 5 together with the results as obtained by using a rat. As shown in Fig. 5, the liposome dispersion liquid of this invention showed an excellent accumulation tendency to an injured portion of blood vessel. By contrast, the Control liposome hardly showed any accumulation tendency to an injured portion of blood vessel.

As apparent from above results, the liposome of this invention is also effective to a hog, which is a large-sized animal and is said to have a blood vessel which is very close to the human blood vessel.

Although not shown in this graph, the liposomes containing a compound having a positively charged portion in the physiological pH other than above-mentioned compound also indicated almost the same excellent filling ratio as those of the Examples.

### (Test 6: Accumulation tendency in organs)

A balloon catheter of 2-French size was introduced through a femoral artery into an SD male rat anesthetized with urethane. Then, the carotid artery of the rat was denuded 5 times thereby forming a model of injured vascular endothelium.

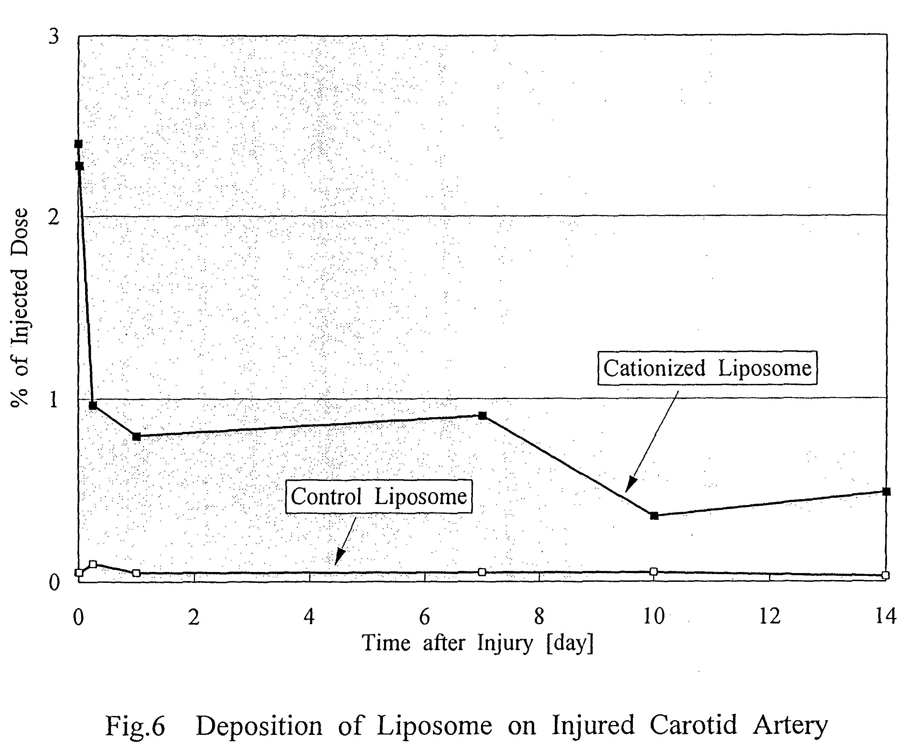

500*µ*l of the liposome dispersion liquid containing 100mM of carboxyfluorescein prepared in accordance with Example 2 was intravenously administered through a femoral vein to each group of SD male rats at various moments, i.e., immediately after the denudation, on 0.1 hour later, 0.5 hour later, 1 hour later, 3 hours later, 6 hours later, and 24 hours later. The samples of the carotid artery were taken out on the third hour of the administration. Then the carboxyfluorescein was extracted from the carotid artery thus taken out, and the fluorescence intensity thereof was measured thereby estimating changes in distribution to the carotid artery of each group. The results are shown in Fig. 6.

As a result, it was observed that the accumulation of the liposome was temporarily lowered when the administration was performed at the moment around one hour later after the denudation, but when the administration was performed thereafter, the ratio of accumulation was not lowered but kept constant. Namely, although the liposome should be administered within one hour from the moment of injury in order to obtain most preferable effect, it is possible to obtain certain degree of effect even if it is administered more than one hour later. Specifically, the accumulation ratio of slightly less than 1% can be maintained, if the drug carrier is administered during a time period of from 6 hours to one week after the moment of injury, and even if the liposome is administered more than one week, it is possible to attain a relatively higher degree of accumulation as compared with that of the Control.

The liposomes containing a compound having a positively charged portion in the physiological pH other than above-mentioned compound also indicated almost the same excellent filling ratio as that of Example 2.

### (Test 7: Inhibition effect of liposome containing various kinds of drug to the thickening of blood vessel)

As in the case of heparin-filled liposome shown in Example 2, a liposome dispersion liquid containing a cyclic AMP(cAMP), captopril, papaverine or forskolin was prepared. As a control, a liposome containing nothing of drug was employed.

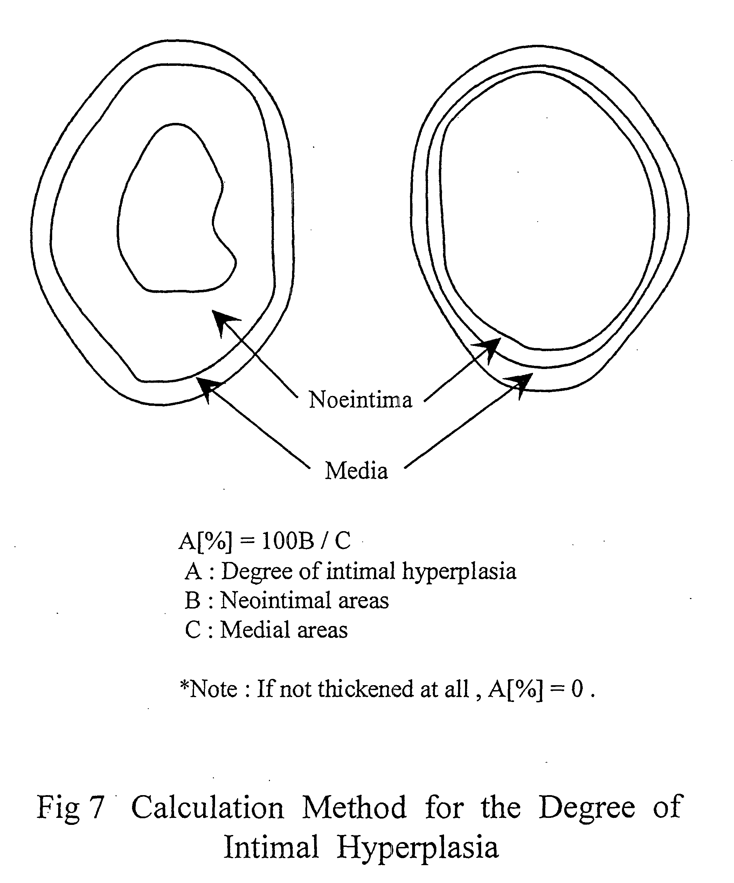

A balloon catheter of 2-French size was introduced through a femoral artery into an SD male rat anesthetized with urethane. Then, the carotid artery of the rat was denuded 4 times thereby forming a model of injured vascular endothelium. Each of drug-filled liposomes was administered the tail vein thereof over 7 days, and then the samples of the carotid artery were collected. Thickening degree of blood vessel was determined by measuring the area ratio in cross-section between the intermediate membrane of the carotid artery and the inner membrane of the carotid artery. The calculating method is illustrated in Fig. 7, and the results are shown in Table 2.

**Table 2**

| Drug filled into liposome | Thickening degree (%) |
|---|---|
| None | 183 |
| Heparin | 131 |
| Theophylline | 146 |
| Cyclic AMP (cAMP) | 133 |
| Captopril | 101 |
| Forskolin | 115 |
| Papaverine | 100 |

As shown in Table 2, the drug-filled liposomes indicated an excellent inhibiting effect to the thickening of blood vessel.

### (Test 8: Distribution in organs)

500*µ*l of the liposome dispersion liquid containing 100mM of carboxyfluorescein prepared in accordance with Example 3 was intravenously administered through a femoral vein to an SD male rat anesthetized with urethane. Carboxyfluorescein was taken out of the organ of the rat, and then measured for a fluorescence intensity thereof thereby measuring the distribution of liposome in the liver of the rat.

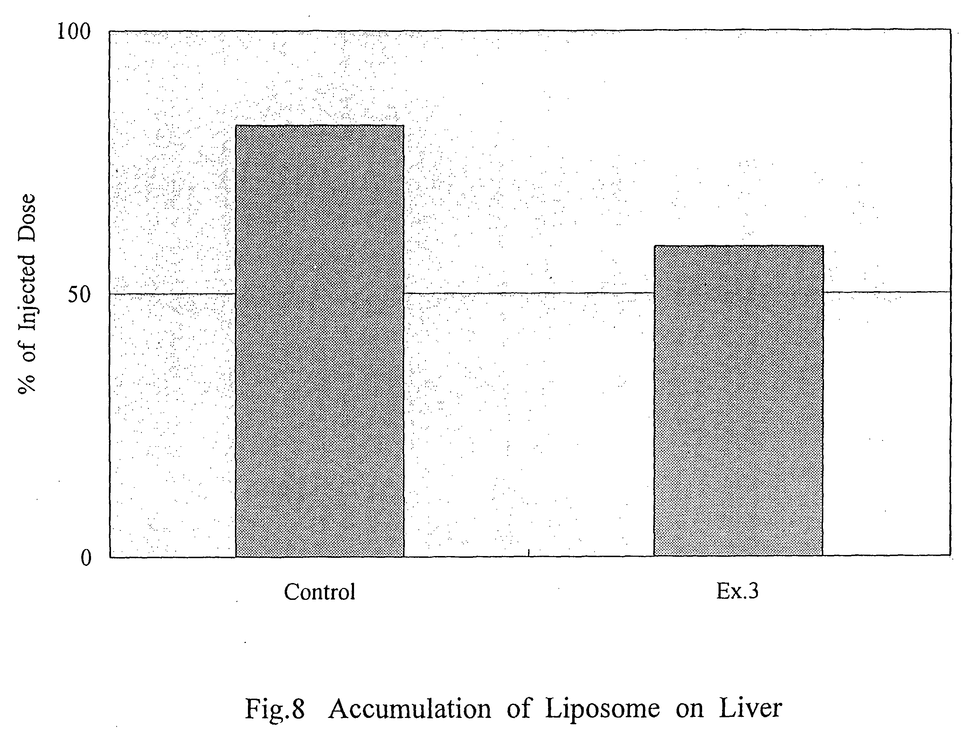

For the purpose of comparison, 6-o-palmitoyl-methyl-D-galactosaminide was employed in place of glucamine palmitate to prepare a liposome dispersion liquid in accordance with the Example 4. The liposome dispersion liquid thus obtained was measured for the distribution thereof in the liver of the rat. Results are shown in Fig 8.

As is clear from the above results, the drug carrier of this invention comprising as a membrane component a closed vehicle is very effective in inhibiting the distribution thereof to a liver as compared with those having a sugar chain as a membrane component. Further, when a hydrophilic polymer is included in a molecular structure of the drug carrier of this invention, the distribution thereof to the liver can be more effectively inhibited. This phenomenon is considered to be attributed to the absence of a sugar chain structure as a surface structure of the membrane component thus avoiding the receptor of the liver, as well as to the presence of a hydrophilic polymer which is also effective in avoiding the receptor of the liver. Therefore, the drug carrier of this invention containing such a membrane component is not likely to be caught by a specific kind of receptor, and would be more excellent in RES-avoiding property if the membrane component includes a hydrophilic polymer.

### (Test 9: Intracorporeal kinetics 3)

A balloon catheter of 2-French size was introduced through a femoral artery into an SD male rat anesthetized with urethane. Then, the carotid artery of the rat was denuded 5 times thereby forming a model of injured vascular endothelium.

Immediately after this denudation, 500*µ*l of the liposome dispersion liquid containing 100mM of carboxyfluorescein prepared in accordance with Example 7 was intravenously administered through a femoral vein to the SD male rat. The samples of the carotid artery were taken out three hours later. The carboxyfluorescein was extracted from the carotid artery taken out in advance, and the fluorescence intensity thereof was measured thereby estimating the distribution of the liposome on the carotid artery.

Likewise, 500*µ*l of the liposome dispersion liquid containing 100mM of carboxyfluorescein prepared respectively in accordance with Example 8 was intravenously administered to the rat through a femoral vein which was not subjected to denudation. Thereafter, the distribution of the liposome to the carotid artery was measured in the same manner as explained above.

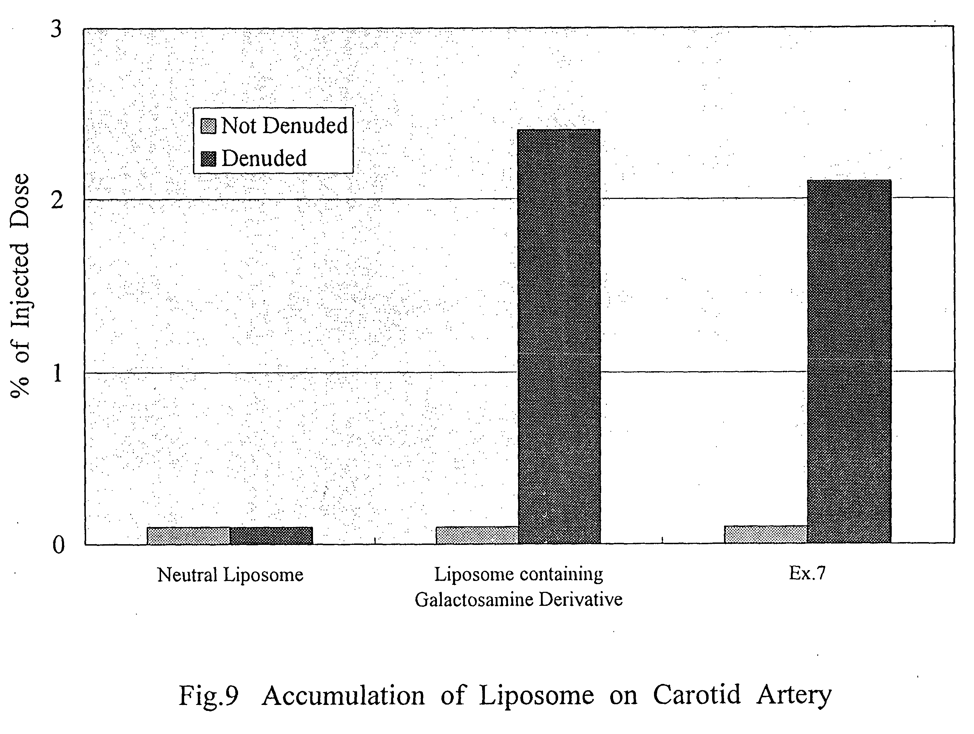

For the purpose of comparison, the distribution of the liposome dispersion liquid to the carotid artery was measured in the same manner as explained above by employing garactosamine derivative in place of N-palmitoyl-3-[(3-aminopropoxy)-poly(oxyethylene)] propylamine (molecular weight: 4000), or by using a liposome dispersion liquid prepared according to Example 8 without employing N-palmitoyl-3-[(3-aminopropoxy)-poly(oxyethylene)] propylamine (molecular weight: 4000). Results are shown in Fig 9.

As is clear from Fig.9, the drug carrier of this invention comprising as a membrane component a closed vehicle is superior in accumulation tendency to an injured portion of vascular endothelium as compared with the drug carrier which does not contain a cationic membrane material. However, if the drug carrier of this invention is applied to vascular endothelium having no injured portion, the accumulation tendency thereof indicated any difference as compared with the Control drug carrier, thus indicating an excellent targeting property of the drug carrier of this invention to an injured portion of vascular endothelium.

### (Test 10: Acute toxicity)

The purpose of this test is know the degree of toxicity of the drug carrier of this invention in comparison with that of the conventional drug carrier. Accordingly, a lethal toxicity test using a rat for investigating the toxicity of the drug carriers of this invention as well as of the conventional drug carriers was conducted without filling a drug in these carriers.

### (Preparation of sample liquid)

### (1) A liposome dispersion liquid of this invention containing 6-o-palmitoyl-methyl-D-galactosaminide (A)

A liposome dispersion liquid containing 6-o-palmitoyl-methyl-D-galactosaminide was obtained in the same manner as in Example 1. However heparin was not incorporated into the liposome dispersion liquid. This liposome dispersion liquid was concentrated by means of ultrafiltration, and diluted as required with a sterilized distilled water thereby obtaining a sample liquid.

### (2) A liposome dispersion liquid of this invention containing 6-o-palmitoyl-methyl-D-galactosaminide and PEG-PE (B)

The procedure of Example 2 was repeated except that heparin was not added to the liposome, thereby obtaining a liposome dispersion liquid containing 6-o-palmitoyl-methyl-D-galactosaminide and PEG-PE. This liposome dispersion liquid was concentrated by means of ultrafiltration, and diluted as required with a sterilized distilled water thereby obtaining a sample liquid.

### (3) A liposome dispersion liquid of this invention containing lysine derivative (C)

The procedure of Example 4 was repeated except that heparin was not added to the liposome, thereby obtaining a liposome dispersion liquid containing lysine. This liposome dispersion liquid was concentrated by means of ultrafiltration, and diluted as required with a sterilized distilled water thereby obtaining a sample liquid.

### (4) A liposome dispersion liquid of this invention containing arginine derivative (D)

The procedure of Example 5 was repeated except that heparin was not added to the liposome, thereby obtaining a liposome dispersion liquid containing arginine. This liposome dispersion liquid was concentrated by means of ultrafiltration, and diluted as required with a sterilized distilled water thereby obtaining a sample liquid.

### (5) A liposome dispersion liquid of this invention containing glucamine palmitate derivative (E)

The procedure of Example 6 was repeated except that heparin was not added to the liposome, thereby obtaining a liposome dispersion liquid containing glucamine palmitate. This liposome dispersion liquid was concentrated by means of ultrafiltration, and diluted as required with a sterilized distilled water thereby obtaining a sample liquid.

### (6) A liposome dispersion liquid of this invention containing N-palmitoyl-3-[(3-aminopropoxy)-poly(oxyethylene)] propylamine (molecular weight: 4000) derivative (F)

The procedure of Example 7 was repeated except that heparin was not added to the liposome, thereby obtaining a liposome dispersion liquid containing N-palmitoyl-3-[(3-aminopropoxy)-poly(oxyethylene)] propylamine (molecular weight: 4000). This liposome dispersion liquid was concentrated by means of ultrafiltration, and diluted as required with a sterilized distilled water thereby obtaining a sample liquid.

### (7) A liposome dispersion liquid of this invention containing galactosamine derivative (G)

For the purpose of comparison, galactosamine was used in place of N-palmitoyl-3-[(3-aminopropoxy)-poly(oxyethylene)] propylamine (molecular weight: 4000), and the procedure of Example 7 was repeated to obtain a neutral liposome dispersion liquid. This liposome dispersion liquid was concentrated by means of ultrafiltration, and diluted as required with a sterilized distilled water thereby obtaining a sample liquid.

### (8) The conventional liposome dispersion liquid (H)

For the purpose of comparison with the liposomes of Examples 1 to 8, a liposome dispersion solution (Control) which contained neither a compound having a positively charged portion, a hydrophobic compound nor a hydrophilic polymer derivative was also prepared in the same manner as in the above-mentioned Examples. This liposome dispersion liquid was concentrated by means of ultrafiltration, and diluted as required with a sterilized distilled water thereby obtaining a sample liquid.

### (9) Physiological salt water (I)

### (Method)

Quarantined 5 weeks old rats were divided into groups, each group consisting of three rats, and 2.0ml of above sample liquid was intraperitoneally administered once to each group of the rats. While, 2.0ml of sterilized distilled water was administered to the group of control.

These rats were carefully observed at least once a day for over 16 days after the administration of the sample liquid to investigate the general conditions of the rats, and the toxic symptom and death of the rats were recorded.

### (Results)

The results are shown in Table 3.

**Table 3**

| Sample liquid | Dosage ml/kg | Deceased population | | | Total of death up to 15th day |
|---|---|---|---|---|---|
| | | up to 1st day | 1st day | 2nd day thereafter | |
| (A) | 0.3 | 0/3 | 0/3 | 0/3 | 0/3 |
| | 1.0 | 0/3 | 0/3 | 0/3 | 0/3 |
| | 3.0 | 0/3 | 0/3 | 0/3 | 0/3 |
| | 10.0 | 0/3 | 0/3 | 0/3 | 0/3 |
| | 0.3 | 0/3 | 0/3 | 0/3 | 0/3 |
| (B) | 1.0 | 0/3 | 0/3 | 0/3 | 0/3 |
| | 3.0 | 0/3 | 0/3 | 0/3 | 0/3 |
| | 10.0 | 0/3 | 0/3 | 0/3 | 0/3 |
| | 0.3 | 0/3 | 0/3 | 0/3 | 0/3 |
| (C) | 1.0 | 0/3 | 0/3 | 0/3 | 0/3 |
| | 3.0 | 0/3 | 0/3 | 0/3 | 0/3 |
| | 10.0 | 0/3 | 0/3 | 0/3 | 0/3 |
| | 0.3 | 0/3 | 0/3 | 0/3 | 0/3 |
| (D) | 1.0 | 0/3 | 0/3 | 0/3 | 0/3 |
| | 3.0 | 0/3 | 0/3 | 0/3 | 0/3 |
| | 10.0 | 0/3 | 0/3 | 0/3 | 0/3 |
| | 0.3 | 0/3 | 0/3 | 0/3 | 0/3 |
| (E) | 1.0 | 0/3 | 0/3 | 0/3 | 0/3 |
| | 3.0 | 0/3 | 0/3 | 0/3 | 0/3 |
| | 10.0 | 0/3 | 0/3 | 0/3 | 0/3 |
| | 0.3 | 0/3 | 0/3 | 0/3 | 0/3 |
| (F) | 1.0 | 0/3 | 0/3 | 0/3 | 0/3 |
| | 3.0 | 0/3 | 0/3 | 0/3 | 0/3 |
| | 10.0 | 0/3 | 0/3 | 0/3 | 0/3 |
| | 0.3 | 0/3 | 0/3 | 0/3 | 0/3 |
| (G) | 1.0 | 0/3 | 0/3 | 0/3 | 0/3 |
| | 3.0 | 0/3 | 0/3 | 0/3 | 0/3 |
| | 10.0 | 0/3 | 0/3 | 0/3 | 0/3 |
| | 0.3 | 0/3 | 0/3 | 0/3 | 0/3 |
| (H) | 1.0 | 0/3 | 0/3 | 1/3 | 1/3 |
| | 3.0 | 0/3 | 0/3 | 1/3 | 1/3 |
| | 10.0 | 0/3 | 1/3 | 1/3 | 2/3 |
| (I) | 10.0 | 0/3 | 0/3 | 0/3 | 0/3 |

As shown in the above test results, deceased population was not admitted during the observation in the drug carriers of this invention. However, in the conventional drug carrier, the weight of the rat was prominently decreased soon after the first day of the drug administration, and most of them were died within 15 days. The cause of this death was confirmed through the observation of the organs to be attributed to the fact that in the case of the conventional drug carrier, the liposome in the drug carrier is most likely to be associated with the hemocyte, or with other liposome thereby forming an aggregation thus giving rise to the formation of embolus in the lungs. In view of these results, it can be said that the drug carrier of this invention is very low in toxicity and excellent in safety as compared with the conventional drug carrier.

Although not shown in the Table 3., the liposomes containing a compound having a positively charged portion in the physiological pH other than above-mentioned compounds also indicated almost the same excellent results as those of the Examples.

As explained above in detail, the drug carrier of this invention is excellent in the filling ratio of neutral or anionic substances as compared with the conventional drug carrier, and also excellent in targeting tendency to the injured portion of vascular endothelium, and in safety.

In view of these features, the drug carrier of this invention carrying therein pharmaceutically allowable substances of pharmacologically active materials, physiologically active materials or diagnostic materials is very effective in diagnosing or treating an injured portion of vascular endothelium. For example, when the drug carrier of this invention is filled with heparin, a polysaccharide, a sulfated polysaccharide, theophylline, a cyclic AMP(cAMP), captopril, papaverine or forskolin, it can be effectively utilized as a blood vessel-thickening inhibiting medicine for controlling migration of smooth muscle cell at the injured region of vascular endothelium.

## Claims

1. A drug carrier which is capable of being selectively accumulated on an injured portion of vascular endothelium, and comprises a compound having a region of positive charge at a physiological pH range which is exposed on the surface of the drug carrier, and a hydrophilic polymer derivative having a hydrophilic polymer region thereof exposed at least partially on the surface of the drug carrier.

2. The drug carrier according to claim 1 wherein said compound comprises at least one cationic residual group on one end of its molecular structure, and a hydrophobic residual group on the other end of the molecular structure.

3. The drug carrier according to claim 1 or claim 2 wherein said hydrophilic polymer derivative is a reaction product of polyethylene glycol with a compound selected from the group consisting of long chain aliphatic acid, long chain alcohol, sterol, polyoxipropylenealkyl and glycerol fatty acid ester.

4. The drug carrier according to any one of claims 1 to 3 which is composed of fine particles having a particle diameter ranging from 0.02 to 250µm.

5. The drug carrier according to any one of the preceding claims which is a macromolecule, a microaggregate, a microglobule, a nanoglobule, a liposome and an emulsion.

6. The drug carrier according to any one of the preceding claims wherein said carrier further comprises a phospholipid or derivative thereof, and/or a lipid other than phospholipid and derivatives of the lipid, and/or a stabilizer, and/or antioxidant, and/or other surface-modifying agents.

7. The drug carrier according to any one of the preceding claims wherein said compound has at least one aliphatic primary, secondary or tertiary amino group, aliphatic quaternary ammonium group, amidino group, aromatic primary, secondary or tertiary amino group, or aromatic quaternary ammonium group, or has a residual group containing at least one hydroxyl group attached thereto in addition to an aliphatic primary, secondary or tertiary amino group, an aliphatic quaternary ammonium group, an amidino group and an aromatic primary, secondary or tertiary amino group, an aromatic quaternary ammonium group.

8. The drug carrier according to any one of claims 1 to 7 wherein said compound is a basic amino acid derivative.

9. The drug carrier according to any one of claims 1 to 8 wherein said compound is an amino sugar.

10. The drug carrier according to claim 9 wherein said amino sugar is glucosamine, galactosamine, mannosamine, neuraminic acid, neuraminic acid ester, another monosaccharide, a free such monosaccharide, or a glycoside of such monosaccharides.

11. The drug carrier according to claim 9 wherein said amino sugar chitin or other oligosaccharide, a polysaccharide, a free such saccharide, or a glycoside of such saccharide.

12. The drug carrier according to any one of the preceding claims further comprising a drug for diagnosing or treating an injured portion of vascular endothelium.

13. The drug carrier according to claim 12 wherein the drug is electrically neutral or anionic.

14. The drug carrier according to claim 12 or claim 13 wherein said drug for diagnosing or treating an injured portion of vascular endothelium is an anti-inflammatory drug, an anti-cancer drug, an enzymatic drug, an antibiotic substance, an antioxidant, a lipid introjection drug, a hormone drug, an angiotensin transformation enzyme-inhibiting drug, a smooth muscle cell-proliferation/migration inhibitor, a platelet aggregation inhibitor, a release inhibitor for a chemical mediator, or a proliferation/migration inhibitor for vascular endothelium.

15. The drug carrier according to claim 12 or claim 13 wherein said drug for diagnosing or treating an injured portion of vascular endothelium is glycosaminoglycan or a derivative thereof.

16. The drug carrier according to claim 12 or claim 13 wherein said drug for diagnosing or treating an injured portion of vascular endothelium is an oligosaccharide, polysaccharide or a derivative thereof.

17. The drug carrier according to claim 12 or claim 13 wherein said drug for diagnosing or treating an injured portion of vascular endothelium is an X-ray contrast medium, a radioisotope marker labelled nuclear medical diagnostic, a nuclear magnetic resonance diagnostic drug, or other intracorporeal diagnostic.

18. The drug carrier according to claim 12 or claim 13 wherein said drug for diagnosing or treating an injured portion of vascular endothelium is heparin, theophylline, a cyclic AMP (cAMP), captopril, papaverine or forskolin.

## Patentansprüche

1. Arzneimittelträger, welcher auf einem geschädigten Bereich des vaskulären Endothels selektiv akkumuliert werden kann und welcher das Folgende umfasst: eine Verbindung mit einer Region positiver Ladung bei einem physiologischen pH-Bereich, die auf der Oberfläche des Arzneimittelträgers frei liegt, und ein hydrophiles Polymerderivat mit einer hydrophilen Polymerregion, die wenigstens teilweise auf der Oberfläche des Arzneimittelträgers frei liegt.

2. Arzneimittelträger gemäß Anspruch 1, wobei die Verbindung wenigstens eine kationische Restgruppe an dem einen Ende seiner Molekülstruktur und eine hydrophobe Restgruppe an dem anderen Ende der Molekülstruktur umfasst.

3. Arzneimittelträger gemäß Anspruch 1 oder Anspruch 2, wobei das hydrophile Polymerderivat ein Reaktionsprodukt von Polyethylenglykol mit einer Verbindung ist, die aus der aus den folgenden Verbindungen bestehenden Gruppe ausgewählt ist: langkettige aliphatische Säure, langkettiger Alkohol, Sterol, Polyoxypropylenalkyl- und Glycerol-Fettsäureester.

4. Arzneimittelträger gemäß irgendeinem der Ansprüche 1 bis 3, welcher aus Feinteilchen mit einem Teilchendurchmesser im Bereich von 0,02 bis 250 µm aufgebaut ist.

5. Arzneimittelträger gemäß irgendeinem der vorhergehenden Ansprüche, welcher ein Makromolekül, ein Mikroaggregat, ein Mikroglobulus, ein Nanoglobulus, ein Liposom und eine Emulsion ist.

6. Arzneimittelträger gemäß irgendeinem der vorhergehenden Ansprüche, wobei der Träger ferner ein Phospholipid oder ein Derivat davon, und/oder ein anderes Lipid außer Phospholipid und ein Derivat des Lipids, und/oder ein Stabilisierungsmittel und/oder ein Antioxidans und/oder andere oberflächenmodifizierende Mittel umfasst.

7. Arzneimittelträger gemäß irgendeinem der vorhergehenden Ansprüche, wobei die Verbindung wenigstens eine aliphatische primäre, sekundäre oder tertiäre Aminogruppe, eine aliphatische quartäre Ammoniumgruppe, eine Amidinogruppe, eine aromatische primäre, sekundäre oder tertiäre Aminogruppe oder aromatische quartäre Ammoniumgruppe aufweist, oder wenigstens eine Restgruppe aufweist, die wenigstens einen daran gebundenen Hydroxylrest zusätzlich zu einer aliphatischen primären, sekundären oder tertiären Aminogruppe, einer aliphatischen quartären Ammoniumgruppe, einer Amidinogruppe und einer aromatischen primären, sekundären oder tertiären Aminogruppe und einer aromatischen quartären Ammoniumgruppe aufweist.

8. Arzneimittelträger gemäß irgendeinem der Ansprüche 1 bis 7, wobei die Verbindung ein basisches Aminosäurederivat ist.

9. Arzneimittelträger gemäß irgendeinem der Ansprüche 1 bis 8, wobei die Verbindung ein Aminozucker ist.

10. Arzneimittelträger gemäß Anspruch 9, wobei der Aminozucker Glucosamin, Galactosamin, Mannosamin, Neuraminsäure, Neuraminsäureester, ein weiteres Monosaccharid, ein freies solches Monosaccharid oder ein Glykosid eines solchen Monosaccharids ist.

11. Arzneimittelträger gemäß Anspruch 9, wobei der Aminozucker Chitin oder ein anderes Oligosaccharid, ein Polysaccharid, ein freies solches Saccharid oder ein Glykosid eines solchen Saccharids ist.

12. Arzneimittelträger gemäß irgendeinem der vorhergehenden Ansprüche, welcher ferner ein Arzneimittel zur Diagnose oder Behandlung eines verletzten Bereichs des vaskulären Endothels umfasst.

13. Arzneimittelträger gemäß Anspruch 12, wobei das Arzneimittel elektrisch neutral oder anionisch ist.

14. Arzneimittelträger gemäß Anspruch 12 oder Anspruch 13, wobei das Arzneimittel zur Diagnose oder Behandlung eines geschädigten Bereichs des vaskulären Endothels ein entzündungshemmendes Arzneimittel, ein Anti-Krebs-Arzneimittel, ein enzymatisches Arzneimittel, eine antibiotische Substanz, ein Antioxidans, ein Arzneimittel zur Verabreichung eines Lipids, ein Hormonarzneimittel, ein Arzneimittel zur Inhibierung von Angiotensin-Transformationsenzym, ein Proliferations-/Migrations-Inhibitor für Zellen der glatten Muskulatur, ein Plättchen-Aggregationsinhibitor, ein Freisetzungsinhibitor für einen chemischen Mediator oder ein Proliferations-/Migrations-Inhibitor für das vaskuläre Endothel ist.

15. Arzneimittelträger gemäß Anspruch 12 oder Anspruch 13, wobei das Arzneimittel zur Diagnose oder Behandlung eines geschädigten Bereichs des vaskulären Endothels ein Glykosaminoglykan oder ein Derivat davon ist.

16. Arzneimittelträger gemäß Anspruch 12 oder Anspruch 13, wobei das Arzneimittel zur Diagnose oder Behandlung eines geschädigten Bereichs des vaskulären Endothels ein Oligosaccharid, Polysaccharid oder ein Derivat davon ist.

17. Arzneimittelträger gemäß Anspruch 12 oder Anspruch 13, wobei das Arzneimittel zur Diagnose oder Behandlung eines geschädigten Bereichs des vaskulären Endothels ein Röntgenstrahl-Kontrastmittel, ein mit Radioisotop-Markern markiertes nuklearmedizinisches diagnostisches Mittel, ein Arzneimittel für die Diagnostik mittels kernmagnetischer Resonanz oder ein anderes intrakorporales diagnostisches Mittel ist.

18. Arzneimittelträger gemäß Anspruch 12 oder Anspruch 13, wobei das Arzneimittel zur Diagnose oder Behandlung eines geschädigten Bereichs des vaskulären Endothels Heparin, Theophyllin, ein cyclisches AMP (cAMP), Captopril, Papaverin oder Forskolin ist.

## Revendications

1. Vecteur pour médicament qui est capable de s'accumuler de manière sélective sur une partie lésée de l'endothélium vasculaire et qui comprend un composé ayant une zone de charge positive dans une plage de pH physiologique qui est exposée sur la surface du vecteur pour médicament et un dérivé de polymère hydrophile dont une zone de polymère hydrophile est exposée au moins partiellement sur la surface du vecteur pour médicament.

2. Vecteur pour médicament selon la revendication 1, dans lequel ledit composé comprend au moins un groupe résiduel cationique à une extrémité de sa structure moléculaire ainsi qu'un groupe résiduel hydrophobe à l'autre extrémité de la structure moléculaire.

3. Vecteur pour médicament selon la revendication 1 ou la revendication 2, dans lequel ledit dérivé de polymère hydrophile est un produit de la réaction de polyéthylène glycol avec un composé choisi dans le groupe comprenant un acide aliphatique à longue chaîne, alcool à longue chaîne, stérol, polyoxypropylène alkyle et ester glycérolique d'acide gras.

4. Vecteur pour médicament selon l'une quelconque des revendications 1 à 3, qui est composé de fines particules ayant un diamètre de particule dans la plage de 0,02 à 250 µm.

5. Vecteur pour médicament selon l'une quelconque des revendications précédentes, qui est une macromolécule, un micro-agrégat, un microglobule, un nanoglobule, un liposome et une émulsion.

6. Vecteur pour médicament selon l'une quelconque des revendications précédentes, dans lequel ledit vecteur comprend en plus un phospholipide ou dérivé de celui-ci et/ou un lipide autre qu'un phospholipide et des dérivés de ce lipide et/ou un stabilisant et/ou un antioxydant et/ou d'autres agents de modification de surface.

7. Vecteur pour médicament selon l'une quelconque des revendications précédentes, dans lequel ledit composé possède au moins un groupe amino primaire, secondaire ou tertiaire aliphatique, un groupe ammonium quaternaire aliphatique, un groupe amidino, un groupe amino primaire, secondaire ou tertiaire aromatique ou un groupe ammonium quaternaire aromatique, ou bien possède un groupe résiduel contenant au moins un groupe hydroxyle qui lui est lié en plus d'un groupe amino primaire, secondaire ou tertiaire aliphatique, d'un groupe ammonium quaternaire aliphatique, d'un groupe amidino, d'un groupe amino primaire, secondaire ou tertiaire aromatique ou d'un groupe ammonium quaternaire aromatique.

8. Vecteur pour médicament selon l'une quelconque des revendications 1 à 7, dans lequel ledit composé est un dérivé d'acide aminé basique.

9. Vecteur pour médicament selon l'une quelconque des revendications 1 à 8, dans lequel ledit composé est un sucre aminé.

10. Vecteur pour médicament selon la revendication 9, dans lequel ledit sucre aminé est la glucosamine, la galactosamine, la mannosamine, l'acide neuraminique, un ester de l'acide neuraminique, un autre monosaccharide, un monosaccharide de ce genre libre ou un glycoside de monosaccharides de ce genre.

11. Vecteur pour médicament selon la revendication 9, dans lequel ledit sucre aminé est la chitine ou un autre oligosaccharide, un polysaccharide, un saccharide de ce genre libre ou un glycoside d'un saccharide de ce genre.

12. Vecteur pour médicament selon l'une quelconque des revendications précédentes, comprenant en plus un médicament pour diagnostiquer ou traiter une partie lésée de l'endothélium vasculaire.

13. Vecteur pour médicament selon la revendication 12, dans lequel le médicament est électriquement neutre ou anionique.

14. Vecteur pour médicament selon la revendication 12 ou la revendication 13, dans lequel ledit médicament pour diagnostiquer ou traiter une partie lésée de l'endothélium vasculaire est un médicament antiinflammatoire, un médicament anticancéreux, un médicament enzymatique, une substance antibiotique, un antioxydant, un médicament d'introjection de lipide, un médicament hormonal, un médicament inhibant l'enzyme de conversion de l'angiotensine, un inhibiteur de la prolifération/migration des cellules des muscles lisses, un inhibiteur de l'agrégation plaquettaire, un inhibiteur de libération d'un médiateur chimique ou un inhibiteur de la prolifération/migration de l'endothélium vasculaire.

15. Vecteur pour médicament selon la revendication 12 ou la revendication 13, dans lequel ledit médicament pour diagnostiquer ou traiter une partie lésée de l'endothélium vasculaire est le glycosaminoglycane ou un dérivé de celui-ci.

16. Vecteur pour médicament selon la revendication 12 ou la revendication 13, dans lequel ledit médicament pour diagnostiquer ou traiter une partie lésée de l'endothélium vasculaire est un oligosaccharide, un polysaccharide ou un dérivé de ceux-ci.

17. Vecteur pour médicament selon la revendication 12 ou la revendication 13, dans lequel ledit médicament pour diagnostiquer ou traiter une partie lésée de l'endothélium vasculaire est un produit de contraste radiologique, un produit de diagnostic marqué par un radio-isotope en médecine nucléaire, un médicament de diagnostic par résonance magnétique nucléaire ou un autre produit de diagnostic intracorporel.

18. Vecteur pour médicament selon la revendication 12 ou la revendication 13, dans lequel ledit médicament pour diagnostiquer ou traiter une partie lésée de l'endothélium vasculaire est l'héparine, la théophylline, un AMP cyclique (AMPc), le captopril, la papavérine ou la forskoline.
